(19) Europäisches Patentamt
European Patent Office
Office européen des brevets

(11) **EP 1 785 105 B1**

(12) **EUROPEAN PATENT SPECIFICATION**

(45) Date of publication and mention
of the grant of the patent:
**25.03.2009 Bulletin 2009/13**

(51) Int Cl.:
*A61B 19/04* (2006.01)    *A41D 19/00* (2006.01)
*C08L 27/06* (2006.01)    *C08K 5/12* (2006.01)

(21) Application number: **07004455.7**

(22) Date of filing: **18.06.2004**

(54) **Disposable gloves**

Wegwerfbare Handschuhe

Gants jettables

(84) Designated Contracting States:
**DE FR GB IT NL**

(30) Priority: **23.06.2003 US 480773**

(43) Date of publication of application:
**16.05.2007 Bulletin 2007/20**

(62) Document number(s) of the earlier application(s) in
accordance with Art. 76 EPC:
**04755751.7 / 1 635 727**

(73) Proprietor: **Medline Industries, Inc.,
Mundelein, IL 60060 (US)**

(72) Inventor: **Hao, Junjie; Room 201, Unit 5
Shijiazhuang City, Hebei ,050081 (CN)**

(74) Representative: **Grünecker, Kinkeldey,
Stockmair & Schwanhäusser
Anwaltssozietät
Leopoldstrasse 4
80802 München (DE)**

(56) References cited:
**US-A- 3 059 241          US-A- 4 082 862
US-A- 6 016 570**

**Description**

FIELD OF THE INVENTION

The present invention relates to gloves and, more specifically, to disposable gloves comprising polyvinyl chloride.

BACKGROUND OF THE INVENTION

Disposable gloves are widely used as a protective measure and have become mandatory in many industries and nearly all medical settings. To allow ease of handling, disposable gloves are made of thin and elastic material to minimize the space between the skin and the glove. One material that has previously enjoyed high usage in manufacturing disposable gloves is latex. Latex is produced from natural rubber trees and is processed to make various products. But many life threatening problems have been associated with the use of latex, especially in situations involving repeated frequent exposure, such as medical practitioners wearing latex gloves. Latex contains proteins, which may contain allergens that affect a certain percentage of the population. Additionally, the increasing amounts of time that latex gloves are worn has resulted in increased occurrences of adverse symptoms.

Synthetic gloves have become the preferred substitute to avoid long term exposure to allergens associated with latex. A problem with existing synthetic gloves is that synthetic gloves are not sufficiently elastic. If gloves are not sufficiently elastic, they do not fit closely with the wrist. Related prior art is disclosed in US 4,082,862, which discloses a two layer PVC glove wherein the two layers comprise different PVC resins.

There is an increasing need to provide a disposable synthetic glove that is more elastic than presently available synthetic gloves.

SUMMARY OF THE INVENTION

According to one disposable glove, which is not part of the invention but important for its understanding, it comprises polyvinyl chloride having a tensile strength before aging of at least about 12 Mpa as measured in accordance with ASTM D 412-98a on a glove sample having a thickness at the hand area of from about 0.08mm to about 0.12mm, and a modulus of elasticity of less than about 2.5 Mpa as measured in accordance with ASTM D 412-98a on a glove sample having a thickness at the hand area of from about0. 08mm to about 0.12mm. The material forming the glove is substantially impermeable to liquid water. The glove may further comprise a plasticizer and an elastomer.

According to another disposable glove, which is not part of the invention but important for its understanding, it comprises polyvinyl chloride having a tensile strength before aging of at least about 12 Mpa as measured in accordance with ASTM D 412-98a on a glove sample having a thickness at the hand area of from about 0.08mm to about 0.12mm, and an elongation of break of greater than about 450% as measured in accordance with ASTM D 412-98a on a glove sample having a thickness at the hand area of from about 0.08mm to about 0.12mm. The material forming the glove is substantially impermeable to liquid water. The glove may further comprise a plasticizer and an elastomer. The elongation of break may be greater than about 500% as measured in accordance with ASTM D 412-98a on a glove sample having a thickness at the hand area of from about0.08mm to about 0.12mm.

According to an embodiment, a disposable glove, comprises a fist polyvinyl chloride resin having a degree of polymerization of from about 1,400 to about 1,700 as measured in accordance with JIS K 6721-77, a second polyvinyl chloride resin having a degree of polymerization of at least 1,750 as measured in accordance with JIS K 6721-77, a plasticizer and an elastomer. The glove may further comprise a viscosity reducer and/or a stabilizing agent.

According to yet another disposable glove, which is not part of the invention but important for its understanding, it comprises polyvinyl chloride having a durometer hardness reading less than 56 on a glove sample having a thickness at the hand area of from about0. 08mm to about 0.12mm, and a compressive strength of less than about 230 Mpa at 0.100 inch deflection on a glove sample having a thickness at the hand area of from about 0.08mm to about 0.12mm. The material forming the glove is substantially impermeable to liquid water.

According to one method, a disposable glove may be formed by providing a first polyvinyl chloride resin having a degree of polymerization of from about 1,400 to about 1,700 as measured in accordance with JIS K 6721-77, a second polyvinyl chloride resin having a degree of polymerization of at least 1,750 as measured in accordance with JIS K 6721-77, a plasticizer and an elastomer. The first polyvinyl chloride, the second polyvinyl chloride, the plasticizer and the elastomer are mixed to form a mixture. The mixture is dipped onto a glove-forming surface and dried so as to form the glove. The mixture may be heated after the glove is formed by dipping the mixture onto a glove- forming surface. One example of a production-line setting that may be used is a dipping time of from about 6 to about 8 seconds onto a glove-forming surface, followed by heating and drying of from about 7 to about 9 minutes at a heating temperature of about 200°C. A viscosity reducer and/or a stabilizing agent may be provided and mixed with the first polyvinyl chloride, the second polyvinyl chloride, the plasticizer and the elastomer to form the mixture.

## BRIEF DESCRIPTION OF THE DRAWINGS

Other advantages of the invention will become apparent on reading the following detailed description and on reference to the drawings in which:

FIG. 1 illustrates a synthetic glove according to one embodiment; and
FIG. 2 illustrates a cross-section generally taken through section line 2-2 of the glove shown in FIG. 1.

## DETAILED DESCRIPTION OF THE ILLUSTRATED EMBODIMENT

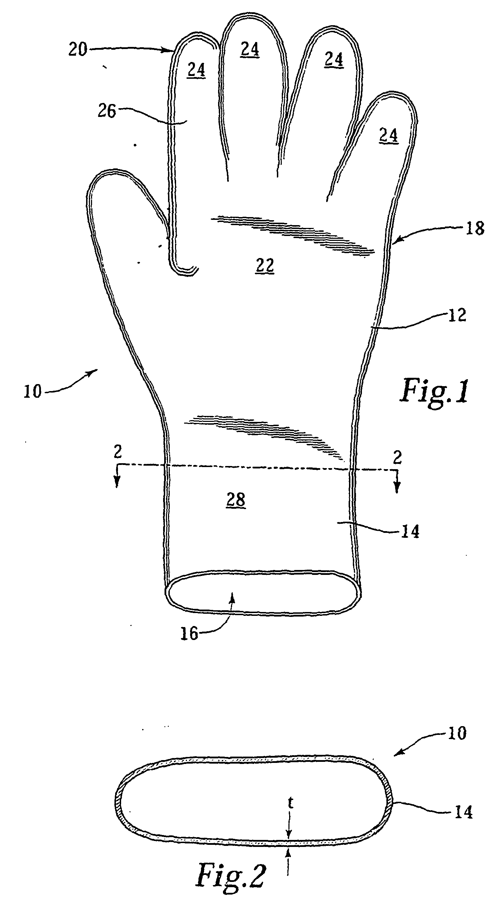

FIG. 1 illustrates a single layer glove 10 comprising a hand area 12 and a wrist area 14 according to one embodiment. The glove 10 of FIG. 1 is substantially impermeable to liquid water and is typically disposable. FIG. 2 is a cross-sectional view of the glove 10 generally taken along section line 2-2 shown in FIG. 1 illustrating a generally uniform thickness t. Although the glove may be of generally uniform thickness t, in one embodiment the hand area 12 has a thickness t from about 0.08mm to about 0.12mm and the wrist area 14 has a thickness t less than about 0.10mm. Thus, the glove may have at least two different thicknesses. In another embodiment, the hand area 12 has a thickness of about 0.10mm and the wrist area 14 has a thickness of about 0.07mm. Thus, the glove in this embodiment has a thickness near or at the wrist area that is less than the thickness at the fingers.

Still referring to FIG. 1, the glove 10 has an open end 16 into which a hand is inserted. Opposite the open end 16 is a distal end 18 that is closed to form a closed end 20. The distal end 18 comprises a primary portion 22 and one or more secondary portions 24 located closer to the closed end 20 than the primary portion 22. In one embodiment, the secondary portion 24 terminates at the closed end 20 and defines a chamber smaller than a chamber defined by the primary portion 22. In the illustrated embodiment, the primary portion 22 includes the hand area 12, whereas one of the secondary portions 24 includes a finger area 26. Similarly, an open-end portion 28 comprises the wrist area 14.

The glove 10 according to one embodiment comprises a suspension-grade polyvinyl chloride (PVC), an emulsion-grade polyvinyl chloride, a plasticizer and an elastomer. To provide improved clarity between the different PVCs, the suspension-grade PVC will also be referred herein as PVC A, while the emulsion-grade PVC will also be referred herein as PVC B. The glove 10 in this embodiment also comprise a viscosity reducer, a stabilizing agent and a color pigment. Tables 1A, 1B, 1C, 1D and 1E set forth examples of components and quantities to form the disposable gloves according to one embodiment.

In this embodiment, the first PVC resin (PVC A) is a suspension-grade homopolymer resin having properties comparable to those set forth in Table 1A below. The PVC A resin is generally characterized as being a micro-suspension grade, having low viscosity, and having a medium degree of polymerization Specifically, the viscosity of PVC A resin was from about 3,000 to about 4,000 cps as determined by ASTM D 1824-90. The degree of polymerization (DP) of PVC A resin is generally from about 1,400 to about 1,700 and, more specifically, from about 1,600 to about 1,700 as measured in accordance with JIS K 6721-77. One example of PVC A resin is a PVC marketed under the name of LP-170g by LG Chemical Ltd. The PVC A resin may be used in paste form. It is contemplated that other resins having these general characteristics may be used in the present invention.

The second PVC resin (PVC B) in this embodiment is a high-polymeric resin having properties comparable to those set forth in Table 1B below. As used herein, the term "high polymeric resin" (i.e., high polymerization-grade resin) is a resin having a degree of polymerization (DP) of at least about 1750, as measured in accordance with JIS K 6721-77. This PVC B resin is generally characterized as being emulsion-grade and having high viscosity. The degree of polymerization (DP) of the PVC B resin is typically from about 1800 to about 2200, and, more specifically, from about 1900 to about 2100 as measured in accordance with JIS K 6721-77. The PVC B resin may used in paste form. The PVC B resin generally has a K-value from about 78 to about 82 as determined by DIN 53726. One example of PVC B resin is marketed under the name of EH-2075 by Hanwha Chemical.

On a proportionate basis, the glove 10 generally comprises a greater amount of the medium polymerization grade resin (PVC A resin) than the amount of the high polymerization grade resin (PVC B resin). It is contemplated that the proportions set forth in Table 1D may be varied. For example, the proportion between the PVC A resin and the PVC B resin is generally from about 40 or 50 to about 75% PVC A resin as compared to PVC B resin and, more specifically, from about 55 to about 65% PVC A resin as compared to PVC B resin. Thus, the glove comprises a ratio of the first polyvinyl chloride resin to the second polyvinyl chloride resin of from about 0.4 or about 0.5 to about 0.75 or, more specifically, a ratio of the first polyvinyl chloride resin to the second polyvinyl chloride resin of from about 0.55 to about 0.65.

According to one embodiment, the glove 10 further comprises a plasticizer. It is contemplated that a number of plasticizers may be used, including those known to one skilled in the art. One example of a desired plasticizer to be used in forming the disposable gloves is diisononyl phthalate (DINP). DINP is desirable because of its balance of several properties. It is contemplated that other plasticizers such as dioctyl phthalate (DOP), diisodecyl phthalate (DIDP), di(2-ethylhexyl) phthalate (DEHP), and combinations thereof may be used. It is also contemplated that DINP may be used in combinations with DOP, DEHP and/or DIDP. Table 1C provides a comparative list of several properties associated with DINP, DOP and DIDP, which is generally referred to as phthalate plasticizers.

It is also contemplated that other plasticizers may be used, such as diethylhexyl adipate (DOA), alone or in combinations with other plasticizers such as those mentioned above. The glove desirably comprises a plasticizer in an amount greater than either the amount of PVC A resin or PVC B resin. In one embodiment, the total amount of the PVC resins is greater than the amount of the plasticizer.

According to one embodiment, the glove 10 further comprises an elastomer. On example of an elastomer that may used is marketed under the name OLICIZER-20N by Aekyung Petrochemical Co., Ltd. This elastomer has a ph of from about 6.5 to about 7.5, a viscosity of from about 2,000 to 3,000 cps at 25°C. The molecular weight of this elastomer is from about 3,000 to 4,000 and has a specific gravity of about 1.10 at 25°C.

It is contemplated that a viscosity reducer, stabilizing agent and color pigment may be added in forming the glove. For example, in one embodiment, the viscosity reducer is TXIB: 2,2-dimethyl-l-(methylethyl)-1,3-propanediyl bis (2-methylpropanoate). It is contemplated that other viscosity reducers may be used. It is contemplated that stabilizing agents known to those skilled in the art made be used. One example is a preparation containing calcium and zinc soap. Color pigment known to those skilled in the art may be added to provide a desired color of the glove.

Table 1D lists components and proportions according to one embodiment, while Table 1E lists some suppliers of these components.

### TABLE1A

| PVC A (Suspension-Grade) Properties | | | | |
|---|---|---|---|---|
| Property/Characteristic | Test Method | Unit | Test Condition | Value |
| Degree of Polymerization (DP) | JIS K 6721-77 | - | 30°C | $1650 \pm 50$ |
| K-value | DIN 53726 | - | - | 78 |
| Apparent Density | ASTM D 1895-90 | $g/cm^3$ | - | $0.34 \pm 0.06$ |
| Brookfield Viscosity (DOP 60phr 100 PVC) | ASTM D 1824-90 | cps | 6 RPM | $3500 \pm 500$ |

### TABLE 1B

| PVC B (Emulsion-Grade) Properties | | | | |
|---|---|---|---|---|
| Property/Characteristic | Test Method | Unit | Test Condition | Value |
| Degree of Polymerization (DP) | JIS K 6721-77 | - | 25°C | $2000 \pm 200$ |
| K-value | JIS 6721 | - | - | 77.4 - 81.0 |
| Apparent Density | JIS 6721 | $g/cm^3$ | - | 0.20 - 0.40 |
| Brookfield Viscosity (DOP 60phr, 100 PVC) | ASTM D 1824-90 | Pa.5 | 25°C | 4.5 |

### TABLE 1C

| Phthalate Property Comparison | | | | | | |
|---|---|---|---|---|---|---|
| Phthalate | Ester Value | Volume Resistivity OHM-cm, 30°C | Viscosity cps, 25°C | Tensile Strength | Modulus 50% $kg/cm^2$ | Molecular Weight |
| DOP | 287 | $2.0 \times 10^3$ | 54 | 225 | 55 | 391 |
| DINP | 268 | $2.0 \times 10^3$ | 59 | 237 | 61 | 419 |

(continued)

| Phthalate Property Comparison | | | | | | |
|---|---|---|---|---|---|---|
| Phthalate | Ester Value | Volume Resistivity OHM-cm, 30°C | Viscosity cps, 25°C | Tensile Strength | Modulus 50% kg/cm$^2$ | Molecular Weight |
| DIDP | 251 | $5.0 \times 10^3$ | 72 | 238 | 72 | 447 |
| Mixture: PVC 100, Plasticizer 50PHR, Stabilizer 1 PHR | | | | | | |

## TABLE 1D

| Component Proportions | |
|---|---|
| Component Raw Materials | Proportion kgs |
| PVC A Resin (Suspension) | 60 |
| PVC B Resin (Emulsion) | 40 |
| Plasticizer | 90 ± 3 |
| Viscosity Reducer | 12 ± 3 |
| Stabilizing Agent | 1. + 2 |
| Color pigment | 2.84 |
| Elastomer | 15 |

## TABLE 1E

| Material/Component | Supplier |
|---|---|
| PVC A Resin LP-170g | LG Chemical Ltd., Yeosu Plant, South Korea |
| PVC B Resin EH-2075 | Hanwha Chemical Corporation, Yeosu, South Korea |
| Plasticizer DINP: diisononyl phthalate | N/A |
| Elastomer OLICIZER-20N | Aekyung Petrochemical Co., Ltd., South Korea |
| Viscosity Reducer TXIB: 2,2-dimethyl-1-(methylethyl)-1,3-propanediyl bis(2-methylpropanoate) | Eastman Chemical Ltd. |
| Stabilizing Agent Preparation containing Calcium and Zinc Soap | N/A |
| Color Pigment white color | N/A |

According to one method, a composition of the materials listed in Table 1E may be prepared as follows. The liquid raw material mix is agitated for about 30 minutes. The solid raw material (e.g., the PVC resin) is added to the liquid raw material and mixed for over 50 minutes. The remaining raw materials are added and the blend is mixed for about 120 minutes so as to obtain a composition viscosity of about 700 cps at a temperature of about 55°C ± 1°. The composition is used to form the gloves in a production-line setting including using dipping times of about 8 seconds onto a glove-forming surface, followed by heating and drying of about 6 minutes at a heating temperature of about 200°C. It is contemplated that other production-line settings may be used such as dipping times of from about 6 to about 8 seconds onto a glove-forming surface, followed by heating and drying of from about 7 to about 9 minutes at a heating temperature of about 200°C.

Some desirable properties of a glove include a tensile strength before aging of greater than 10 Mpa and typically greater than about 12 Mpa for a sample thickness of from about 0.08 to about 0.12mm as measured in accordance with ASTM D 412-98a. It is more desirable to have a tensile strength before aging of greater than 10 Mpa and typically greater than about 13 or 14 Mpa for a sample thickness of from about 0.08 to about 0.12mm as measured in accordance with ASTM D 412-98a.

The elongation break of a glove is generally greater than 400% and typically greater than 450% or 500% for a sample thickness at the hand area of from about 0.08 to about 0.12mm as measured in accordance with ASTM D 412-98a. The elongation break of a glove may even be greater than 525 or 550% for a sample thickness at the hand area of from about 0.08 to about 0.12mm as measured in accordance with ASTM D 412-98a.

It is also desirable to have a 100% stress at a definite elongation of less than 4.5 Mpa for a sample thickness at the hand area of from about 0.08 to about 0.12mm as measured in accordance with ASTM D 412-98a. It is also desirable to have a 100% stress at a definite elongation of less than 4.0 Mpa or less than 3.5 MPa for a sample thickness at the hand area of from about 0.08 to about 0.12mm as measured in accordance with ASTM D 412-98a.

It is also desirable to have a 100% load at a definite elongation of less than 2.8 N for a sample thickness at the hand area of from about 0.08 to about 0.12mm as measured in accordance with ASTM D 412-98a. It is also desirable to have a 100% load at a definite elongation of less than 2.5 N or less than 2.25 N for a sample thickness at the hand area of from about 0.08 to about 0.12mm as measured in accordance with ASTM D 412-98a.

The gloves have desirable numbers when performing a durometer hardness test. A durometer hardness determines the indentation hardness of the material. It is believed that the durometer hardness is indicative of softness and that the lower the durometer hardness, the softer the glove. The durometer hardness test is defined herein by the following steps:

1. Die cut, square specimens, approximately 50,8 mm x 50,8 mm (2"x 2") (width x length) are prepared from randomly chosen gloves, so that they can be stacked to a thickness of approximately 6,35 mm (0.25 inch).

2. The individual samples are evenly stacked on top of each other. The outside of each of the square glove specimens faces upward and the inside faces downwards. The total thickness of the stack is measured and recorded in accordance with ASTM D3767-01 Standard Practice for Rubber-Measurement of Dimensions, Method A.

3. The stacked specimens are conditioned at 23 C and 50% R. H. (relative humidity) for a minimum of 40 hours prior to testing.

4. A calibrated Type A Durometer is used to determine the hardness on the top layer of the stacked specimens, based upon ASTM D2240-03 Standard Test Method for Rubber Property-Durometer Hardness.

The gloves generally have a durometer hardness in accordance with the above procedure of less than 57. More specifically, the gloves of the present invention generally have a durometer hardness in accordance with the above procedure of less than 55 or 53.

The gloves also have desirable numbers when performing a compressive strength test. Compressive strength measures the force in pounds necessary to compress the material to a specified deflection. It is also believed that compressive strength is indicative of softness and that the lower the compressive strength, the softer the glove. The compressive strength test is defined herein by the following steps:

1. Die cut, square specimens, approximately 50,8 mm x 50,8 mm (2"x 2") (width x length) are prepared from randomly chosen gloves, so that they can be stacked to a thickness of approximately 6,35 mm (0.25 inch).

2. The individual samples are evenly stacked on top of each other. The outside of each of the square glove specimens faces upward and the inside faces downwards. The total thickness of the stack is measured and recorded in accordance with ASTM D3767-01 Standard Practice for Rubber-Measurement of Dimensions, Method A.

3. The specimens are conditioned at 23° C and 53% R. H. (relative humidity) for a minimum of 40 hours prior to testing.

4. The stacked specimen is placed on a flat stationary platen of a suitable compression tester.

5. A flat, metal, 19,05 mm (0. 75") diameter presser foot is attached to the upper movable platen of the compression tester.

6. The specimen stack is compressed a total of approximately 5,08mm (0.2 inch), at a crosshead speed of 1,27 mm/minute (0.050 inch/minute).

7. The force in pounds necessary to compress the specimen stack to 1,27mm; 1,905 mm; 2,54 mm; 3,175 mm; 3,81 mm; 4,445 mm (0.050 inch, 0.075 inch, 0.100 inch, 0.125 inch, 0.150 inch, and 0.175 inch) are recorded.

The gloves generally have a compressive strength in accordance with the above procedure at 2,54 mm (0.100 inch) deflection of less than about 1,02 kN (230 lbs). More specifically, the gloves of the present invention generally have a compressive strength in accordance with the above procedure at 2,54 mm (0.100 inch) deflection of less than about 956 or 890 N (215 or 200lbs).

EXAMPLES

Example 1

To show the improved properties of the inventive gloves, several properties were tested in accordance with ASTM D 412-98a. Tables 2 and 3 (located below), which were tested by a first laboratory, compiled several properties from a Comparative Glove 1 and an Inventive Glove1. The measurements were in accordance with ASTM D412-98a and were applied to a sample having a length of about 40 mm and a thickness at the hand area of about 0.10 mm. The stretch speed was 500 mm/min.

Comparative Glove 1 was marketed by Medline as a powder-free vinyl synthetic glove and is referred to as MDS 192075. Comparative Glove 1 included polyvinyl chloride (PVC), di-octyl-phthalate (DOP) or diethylhexyl adipate (DOA), 2,2-dimethyl-1-(methylethyl)-1, 3-propanediyl bis (2-methylpropanoate) (TXIB), stabilizer, epoxidized soybean oil and polyurethane (PU) emulsion. The PVC resin used in Comparative Glove 1 was a LP 170G resin having a degree of polymerization between 1400 and 1700. Inventive Glove 1 had the composition and quantities as set forth above in Tables 1 D and 1 E.

Tables 2 and 3 illustrate physical property sets for nineteen samples manufactured in accordance with the above process for Comparative Glove 1 and twenty samples manufactured in accordance with the above process for Inventive Glove 1, respectively.

**Table 2**

| Comparative Glove 1 | | | | | | | | |
|---|---|---|---|---|---|---|---|---|
| Sample No. | Load at 300% Elongation (N) | Stress at 300% Elongation (Mpa) | Max Load (N) | Tensile Strength (Mpa) | Elasticity Modulus (Mpa) | Elongation of Break (%) | Stress at 100% Elongation (Mpa) | Load at 100% Elongation (N) |
| 1 | 7.549 | 12.58 | 10.45 | 17.41 | 3.91 | 416.5 | 5.09 | 3.054 |
| 2 | 7.598 | 12.66 | 10.83 | 18.06 | 3.88 | 425.2 | 5.06 | 3.036 |
| 3 | 7.246 | 12.08 | 10.95 | 18.24 | 3.73 | 458.9 | 4.69 | 2.812 |
| 4 | 7.147 | 11.91 | 8.26 | 13.77 | 3.69 | 350.7 | 4.87 | 2.920 |
| 5 | 7.254 | 11.52 | 11.17 | 17.73 | 3.41 | 468.0 | 4.68 | 2.951 |
| 6 | 7.888 | 12.52 | 10.37 | 16.45 | 3.82 | 397.6 | 5.23 | 3.295 |
| 7 | 7.429 | 11.79 | 10.22 | 16.22 | 3.59 | 416.3 | 4.73 | 2.982 |
| 8 | 7.004 | 11.67 | 9.99 | 16.66 | 3.54 | 432.8 | 4.72 | 2.830 |
| 9 | 7.344 | 11.66 | 10.09 | 16.02 | 3.61 | 420.2 | 4.62 | 2.911 |
| 10 | 7.241 | 12.07 | 9.69 | 16.15 | 3.67 | 402.9 | 4.91 | 2.946 |
| 11 | 7.321 | 12.20 | 10.33 | 17.22 | 3.66 | 427.1 | 5.08 | 3.049 |
| 12 | 7.237 | 12.06 | 9.70 | 16.16 | 3.65 | 413.5 | 4.91 | 2.946 |
| 13 | 7.237 | 12.06 | 9.75 | 16.25 | 3.70 | 415.0 | 4.89 | 2.933 |

(continued)

| Comparative Glove 1 | | | | | | | | |
|---|---|---|---|---|---|---|---|---|
| Sample No. | Load at 300% Elongation (N) | Stress at 300% Elongation (Mpa) | Max Load (N) | Tensile Strength (Mpa) | Elasticity Modulus (Mpa) | Elongation of Break (%) | Stress at 100% Elongation (Mpa) | Load at 100% Elongation (N) |
| 14 | 7.1 12 | 12.48 | 9.55 | 16.75 | 3.82 | 409.1 | 5.24 | 2.987 |
| 15 | 7.223 | 12.04 | 10.79 | 17.98 | 3.66 | 459.2 | 4.91 | 2.946 |
| 16 | 7.397 | 12.33 | 10.14 | 16.90 | 3.72 | 416.9 | 5.08 | 3.049 |
| 17 | 6.875 | 12.06 | 9.00 | 15.80 | 3.59 | 403.3 | 5.04 | 2.871 |
| 18 | 7.496 | 12.49 | 10.29 | 17.16 | 3.82 | 413.5 | 5.07 | 3.045 |
| 19 | 6.607 | 11.59 | 9.34 | 16.38 | 3.57 | 431.3 | 4.60 | 2.621 |
| Average | 7.274 | 12.09 | 9.84 | 16.36 | 3.70 | 411.0 | 4.91 | 2.952 |

**Table 3**

| Inventive Glove 1 | | | | | | | | |
|---|---|---|---|---|---|---|---|---|
| Sample No. | Load at 300% Elongation (N) | Stress at 300% Elongation (Mpa) | Max Load (N) | Tensile Strength (Mpa) | Elasticity Modulus (Mpa) | Elongation of Break (%) | Stress at 100% Elongation (Mpa) | Load at 100% Elongation (N) |
| 1 | 5.192 | 8.65 | 8.504 | 14.17 | 2.59 | 487.7 | 3.49 | 2.094 |
| 2 | 5.058 | 8.43 | 7.397 | 12.33 | 2.52 | 442.1 | 3.33 | 2.000 |
| 3 | 5.259 | 8.35 | 8.321 | 13.21 | 2.49 | 477.0 | 3.30 | 2.076 |
| 4 | 5.232 | 8.30 | 8.286 | 13.15 | 2.47 | 478.1 | 3.33 | 2.098 |
| 5 | 5.062 | 8.44 | 7.879 | 13.13 | 2.45 | 463.2 | 3.39 | 2.036 |
| 6 | 5.129 | 8.14 | 8.071 | 12.81 | 2.38 | 482.4 | 3.27 | 2.062 |
| 7 | 5.277 | 8.79 | 8.487 | 14.14 | 2.56 | 486.2 | 3.62 | 2.170 |
| 8 | 5.379 | 8.97 | 8.317 | 13.86 | 2.50 | 463.1 | 3.66 | 2.196 |
| 9 | 5.121 | 8.53 | 7.540 | 12.57 | 2.59 | 445.0 | 3.41 | 2.045 |
| 10 | 5.112 | 8.52 | 7.933 | 13.22 | 2.39 | 466.8 | 3.57 | 2.143 |
| 11 | 5.009 | 8.35 | 8.348 | 13.91 | 2.48 | 502.9 | 3.29 | 1.973 |
| 12 | 5.250 | 8.75 | 8.402 | 14.00 | 2.58 | 483.9 | 3.53 | 2.121 |
| 13 | 5.013 | 8.36 | 7.647 | 12.75 | 2.49 | 461.4 | 3.26 | 1.955 |
| 14 | 5.152 | 8.59 | 7.080 | 11.80 | 2.64 | 416.8 | 3.36 | 2.018 |
| 15 | 5.393 | 8.99 | 8.210 | 13.68 | 2.63 | 458.1 | 3.54 | 2.125 |
| 16 | 5.317 | 8.86 | 6.223 | 10.37 | 2.74 | 355.0 | 3.59 | 2.152 |
| 17 | 5.165 | 8.61 | 8.121 | 13.53 | 2.51 | 482.0 | 3.31 | 1.987 |
| 18 | 4.817 | 8.45 | 7.567 | 13.28 | 2.50 | 463.6 | 3.34 | 1.906 |
| 19 | 5.286 | 8.81 | 7.223 | 12.04 | 2.54 | 420.4 | 3.53 | 2.121 |
| 20 | 5.098 | 8.94 | 8.049 | 14.12 | 2.67 | 466.7 | 3.54 | 2.018 |
| Average | 5.166 | 8.59 | 7.880 | 13.10 | 2.54 | 460.1 | 3.43 | 2.065 |

As shown in Tables 2 and 3, Inventive Glove 1 had a much more desirable average elongation of break than the Comparative Glove 1. Compare 460% of Inventive Glove 1 and 411 % of Comparative Glove 1. Additionally, Inventive Glove 1 had a much more desirable average elasticity modulus than Comparative Glove 1. Compare 2.54 Mpa of Inventive Glove 1 and 3.70 Mpa of Comparative Glove 1. The desirable elasticity modulus and elongation of break did not result in an undesirable tensile strength in Inventive Glove 1. The average tensile strength before aging of Inventive Glove 1 was 13.1 Mpa and the average tensile strength before aging of Comparative Glove 1 was 16.7 Mpa. Thus, Inventive Glove 1 had a surprising balance of desirable properties.

Example 2

To show the improved properties of the inventive gloves, several properties were tested in accordance with ASTM D 412-98a. Tables 4-6 (located below), which were tested by a second laboratory, compiled several properties from Comparative Gloves 1 and 2, and Inventive Glove 1. The measurements were in accordance with ASTM D 412-98a and were applied to a sample having a length of about 40 mm and a thickness at the hand area of about 0.10 mm. The stretch speed was 500 mm/min.

Comparative Glove 1 was marketed by Medline as a powder-free vinyl synthetic glove as MDS 192075. Comparative Glove 1 included polyvinyl chloride (PVC), di-octyl-phthalate (DOP) or diethylhexyl adipate (DOA), 2,2-dimethyl-1-(methylethyl)-1,3-propanediyl bis(2-methylpropanoate) (TXIB), stabilizer, epoxidized soybean oil and polyurethane (PU) emulsion. The PVC resin used in Comparative Glove 1 was a LP 170G resin having a degree of polymerization between 1400 and 1700.

Comparative Glove 2 was marketed under MediGuard® Advantage and described as a vinyl synthetic, powder-free, examination glove under the number MSV 502. Comparative Glove 2 included polyvinyl chloride (PVC), di-octyl-phthalate (DOP), 2,2-dimethyl-1-(methylethyl)-1,3-propanediyl bis(2-methylpropanoate) (TXIB), stabilizer, epoxidized soybean oil, polyurethane (PU) emulsion and HCC25108 yellow pigment. The PVC resin used in Comparative Glove 2 was a LP 170G resin having a degree of polymerization between 1400 and 1700. The proportions of Comparative Glove 2 are as follows: 100 PVC, 88 DEHP, 12 TXIB, 1 stabilizer and 2.34 color pigment.

Inventive Glove 1 had the composition and quantities as set forth above in Tables 1D and 1E.

Tables 4-6 illustrate physical property sets for twenty samples manufactured in accordance with the above process for the Comparative Gloves 1 and 2, and Inventive Glove 1, respectively.

**Table 4**

EP 1 785 105 B1

| Comparative Glove 1 | | | | | | | | |
|---|---|---|---|---|---|---|---|---|
| Sample No. | Load at 300% Elongation (N) | Stress at 300% Elongation (Mpa) | Max Load (N) | Tensile Strength (MPa) | Elasticity Modulus (MPa) | Elongation of Break (%) | Stress at 100% Elongation (Mpa) | Load at 100% Elongation (N) |
| 1 | 6.759 | 11.26 | 10.370 | 17.28 | 3.17 | 561.6 | 4.97 | 2.982 |
| 2 | 6.456 | 10.76 | 9.348 | 15.58 | 3.02 | 448.6 | 4.65 | 2.781 |
| 3 | 6.393 | 10.65 | 9.429 | 15.71 | 3.00 | 513.2 | 4.50 | 2.701 |
| 4 | 6.067 | 10.11 | 9.768 | 16.28 | 2.95 | 485.1 | 4.32 | 2.589 |
| 5 | 6.174 | 10.29 | 9.143 | 15.24 | 3.05 | 447.9 | 4.31 | 2.585 |
| 6 | 6.121 | 10.74 | 9.371 | 16.44 | 3.04 | 465.8 | 4.61 | 2.625 |
| 7 | 6.103 | 10.17 | 9.500 | 15.83 | 2.91 | 585.8 | 4.31 | 2.585 |
| 8 | 6.335 | 10.56 | 9.563 | 15.94 | 2.96 | 465.2 | 4.47 | 2.683 |
| 9 | 6.156 | 10.26 | 10.070 | 16.78 | 3.01 | 489.0 | 4.29 | 2.571 |
| 10 | 6.402 | 11.86 | 9.558 | 17.70 | 3.34 | 466.1 | 4.99 | 2.696 |
| 11 | 6.179 | 10.30 | 9.214 | 15.36 | 3.00 | 447.6 | 4.35 | 2.612 |
| 12 | 6.379 | 10.63 | 10.830 | 18.05 | 3.10 | 506.2 | 4.51 | 2.705 |
| 13 | 6.107 | 10.18 | 9.634 | 16.06 | 2.95 | 530.6 | 4.30 | 2.580 |
| 14 | 6.424 | 11.27 | 9.598 | 16.86 | 3.28 | 504.4 | 4.78 | 2.723 |
| 15 | 6.201 | 10.33 | 8.031 | 13.39 | 2.91 | 473.5 | 4.42 | 2.652 |
| 16 | 5.982 | 9.97 | 8.960 | 14.93 | 2.80 | 585.6 | 4.29 | 2.576 |
| 17 | 6.156 | 11.40 | 7.808 | 14.46 | 3.16 | 467.4 | 4.84 | 2.612 |
| 18 | 6.125 | 10.21 | 9.214 | 15.36 | 2.89 | 550.6 | 4.37 | 2.621 |
| 19 | 6.424 | 10.71 | 9.491 | 15.82 | 3.12 | 656.8 | 4.57 | 2.741 |
| 20 | 6.179 | 10.30 | 9.580 | 15.97 | 2.97 | 525.0 | 4.36 | 2.616 |
| Average | 6.256 | 10.60 | 9.424 | 15.95 | 3.03 | 508.8 | 4.51 | 2.662 |

**Table 5**

| Comparative Glove 2 | | | | | | | | |
|---|---|---|---|---|---|---|---|---|
| Sample No. | Load at 300% Elongation (N) | Stress at 300% Elongation (Mpa) | Max Load (N) | Tensile Strength (Mpa) | Elasticity Modulus (Mpa) | Elongation of Break (%) | Stress at 100% Elongation (Mpa) | Load at 100% Elongation (N) |
| 1 | 5.768 | 9.61 | 8.853 | 14.75 | 2.68 | 560.3 | 4.13 | 2.478 |
| 2 | 5.554 | 8.82 | 9.661 | 15.33 | 2.54 | 528.3 | 3.73 | 2.348 |
| 3 | 5.518 | 9.20 | 9.732 | 16.22 | 2.63 | 519.8 | 3.99 | 2.393 |
| 4 | 5.696 | 9.49 | 10.21 | 17.02 | 2.74 | 619.7 | 4.02 | 2.411 |
| 5 | 5.571 | 9.29 | 9.237 | 15.39 | 2.69 | 556.6 | 3.90 | 2.339 |
| 6 | 5.723 | 9.54 | 9.201 | 15.33 | 2.64 | 475.4 | 4.06 | 2.438 |
| 7 | 6.196 | 10.33 | 9.817 | 16.36 | 3.01 | 482.7 | 4.36 | 2.616 |
| 8 | 5.594 | 9.32 | 9.018 | 15.03 | 2.77 | 586.8 | 3.90 | 2.339 |
| 9 | 5.732 | 9.10 | 9.143 | 14.51 | 2.61 | 562.1 | 3.85 | 2.424 |
| 10 | 5.375 | 8.96 | 8.603 | 14.34 | 2.57 | 461.1 | 3.90 | 2.339 |
| 11 | 5.638 | 9.40 | 8.879 | 14.80 | 2.65 | 469.8 | 4.11 | 2.464 |
| 12 | 5.920 | 9.87 | 8.973 | 14.96 | 2.84 | 468.3 | 4.20 | 2.522 |
| 13 | 5.500 | 9.17 | 9.308 | 15.51 | 2.68 | 590.4 | 3.81 | 2.286 |
| 14 | 5.379 | 8.97 | 8.161 | 13.60 | 2.59 | 555.9 | 3.73 | 2.237 |
| 15 | 5.777 | 9.63 | 9.527 | 15.88 | 2.75 | 484.0 | 4.09 | 2.455 |
| 16 | 5.679 | 9.46 | 8.103 | 13.50 | 2.64 | 509.7 | 3.96 | 2.375 |
| 17 | 5.527 | 9.21 | 9.402 | 15.67 | 2.66 | 593.2 | 3.84 | 2.304 |
| 18 | 5.567 | 9.28 | 8.817 | 14.69 | 2.72 | 471.9 | 3.92 | 2.353 |
| 19 | 5.571 | 9.29 | 8.411 | 14.02 | 2.62 | 513.5 | 3.91 | 2.348 |
| 20 | 5.487 | 9.14 | 9.420 | 15.70 | 2.63 | 502.4 | 3.83 | 2.299 |
| Average | 5.639 | 9.35 | 9.124 | 15.13 | 2.68 | 525.6 | 3.96 | 2.388 |

**Table 6**

| Inventive Glove 1 | | | | | | | | |
|---|---|---|---|---|---|---|---|---|
| Sample No. | Load at 300% Elongation (N) | Stress at 300% Elongation (Mpa) | Max Load (N) | Tensile Strength (Mpa) | Elasticity Modulus (Mpa) | Elongation of Break (%) | Stress at 100% Elongation (Mpa) | Load at 100% Elongation (N) |
| 1 | 4.969 | 8.28 | 7.665 | 12.78 | 2.30 | 600.2 | 3.53 | 2.116 |
| 2 | 5.741 | 8.70 | 9.138 | 13.85 | 2.48 | 634.1 | 3.69 | 2.433 |
| 3 | 5.491 | 7.63 | 9.451 | 13.13 | 2.15 | 506.4 | 3.32 | 2.388 |
| 4 | 5.125 | 8.54 | 8.795 | 14.66 | 2.50 | 634.2 | 3.68 | 2.205 |
| 5 | 4.594 | 6.38 | 7.460 | 10.36 | 1.80 | 582.5 | 2.83 | 2.040 |
| 6 | 5.313 | 8.05 | 9.232 | 13.99 | 2.32 | 628.6 | 3.49 | 2.304 |
| 7 | 5.473 | 8.29 | 9.527 | 14.43 | 2.40 | 529.4 | 3.55 | 2.344 |
| 8 | 5.254 | 7.96 | 9.134 | 13.84 | 2.29 | 538.4 | 3.42 | 2.259 |
| 9 | 4.973 | 7.54 | 8.232 | 12.47 | 2.10 | 505.4 | 3.29 | 2.174 |
| 10 | 5.103 | 8.50 | 9.170 | 15.28 | 2.41 | 543.1 | 3.71 | 2.223 |
| 11 | 5.112 | 8.52 | 8.518 | 14.20 | 2.46 | 500.4 | 3.63 | 2.179 |
| 12 | 4.759 | 7.21 | 5.598 | 13.03 | 2.09 | 616.7 | 3.04 | 2.009 |
| 13 | 4.473 | 6.78 | 7.875 | 11.93 | 1.91 | 623.1 | 2.92 | 1.924 |
| 14 | 4.536 | 7.56 | 8.076 | 13.46 | 2.21 | 612.2 | 3.17 | 1.902 |
| 15 | 4.554 | 7.59 | 8.085 | 13.47 | 2.21 | 600.4 | 3.24 | 1.946 |
| 16 | 4.714 | 7.86 | 8.335 | 13.89 | 2.32 | 514.3 | 3.33 | 1.996 |
| 17 | 4.357 | 7.26 | 7.768 | 12.95 | 2.09 | 639.4 | 3.16 | 1.897 |
| 18 | 4.571 | 7.62 | 7.826 | 13.04 | 2.10 | 583.5 | 3.30 | 1.978 |
| 19 | 4.754 | 7.92 | 8.009 | 13.35 | 2.29 | 507.5 | 3.37 | 2.022 |
| 20 | 4.656 | 7.76 | 8.509 | 14.18 | 2.25 | 680.1 | 3.36 | 2.013 |
| Average | 4.926 | 7.80 | 8.470 | 13.41 | 2.23 | 579.0 | 3.35 | 2.118 |

As shown in Tables 4-6, Inventive Glove 1 had a much more desirable average elongation of break than the Comparative Gloves 1 and 2. Compare 579% of Inventive Glove 1 in Table 6 and 509 and 526% of Comparative Gloves 1 and 2 in Tables 4 and 5, respectively. Additionally, Inventive Glove 1 had a much more desirable average elasticity modulus than Comparative Gloves 1 and 2. Compare 2.23 Mpa of Inventive Glove 1 in Table 6 and 3.03 and 2.68 Mpa of Comparative Gloves 1 and 2 in Tables 4 and 5, respectively. The desirable elasticity modulus and elongation of break did not result in an undesirable tensile strength in Inventive Glove 1. The average tensile strength before aging of Inventive Glove 1 was 13.4 Mpa and the average tensile strength before aging of Comparative Gloves 1 and 2 were 16.0 and 15.1 in Tables 4 and 5, respectively.

Example 3

A test was also conducted to compare the restore rates of Comparative Glove 1 and Inventive Glove 1. The Comparative Glove 1 and Inventive Glove 1 are the same as described above in Example 1. Tests were done on Comparative Glove 1 and Inventive Glove 1 samples having a length of 40mm and a width of 6mm. Using an extension rate of 500mm/min, each sample was pulled to a stretch length of 80mm, then held at stretched elongation for 30 seconds and then unloaded. After 60 seconds, the elongation length (*i.e.*, the post-stretch unloaded length) was measured. The restore rate was then calculated according to the following formula (Equation A):

$$\text{Eq.A (restore rate)} = \frac{\text{Elongation length - Original length}}{\text{Original length}} \times 100\%$$

The results of the restore rate test for Comparative Example 1 and Inventive Example 1 are tabulated in Table 7.

**Table 7**

| Sample No. | Inventive Glove 1 | | Comparative Glove 2 | |
|---|---|---|---|---|
| | E-L | R% | E-L | R% |
| 1 | 42 | 95 | 44 | 90 |
| 2 | 42 | 95 | 44 | 90 |
| 3 | 42 | 95 | 43.5 | 91.25 |
| 4 | 42 | 95 | 44 | 90 |
| 5 | 42 | 95 | 44 | 90 |
| 6 | 42.5 | 93.75 | 44 | 90 |
| 7 | 42 | 95 | 44 | 90 |
| 8 | 42 | 95 | 44 | 90 |
| 9 | 42 | 95 | 44 | 90 |
| 10 | 42.5 | 93.75 | 43.5 | 91.25 |
| E-L = Elongation length; R% = Restore rate | | | | |

Inventive Glove 1 had a better average restore rate than that in Comparative Glove 1. Compare restore rates of 95% and 93.75% of Inventive Glove 1 samples and restore rates of 90% and 91.25% of Comparative Glove 1 samples. A higher restore rate is more desirable if a closely fitting glove is needed. The high restore rate allows the glove to be pulled over larger portions of a hand, such as the palm and knuckles of the hand, and then return to closer to its original shape, resulting in closer fitting at, for example, the wrist and fingers. Some application where a closely fitting glove is desired is where tactile sense is important. Thus, Inventive Glove 1 has a desirable restore rate of Table 7, in combination with the low elasticity of modulus discussed shown in Tables 3 and 6 above. Such a combination assists in preventing or inhibiting glove sag and a loose fit.

Example 4

A test was also conducted to compare the durometer hardness readings of Comparative Gloves 3-5 and Inventive Glove 2. Inventive Glove 2 was a commercial embodiment with the same composition and quantities as set forth above in Tables 1D and 1E. Inventive Glove 2 had a thickness at the hand area of about 0.11 mm. Comparative Glove 3 was marketed by Ansell as its MICRO-TOUCH®ELITE® Product No. 3092. Comparative Glove 4 was marketed by Kimberly-Clark as its SAFEShIN®Synthetic Plus Product No. 50032. Comparative Glove 5 was marketed by Allegiance as its ESTEEM™ Product No. 8882. The durometer hardness was determined for Inventive Glove 2 and Comparative Gloves 3-5 in accordance with the procedure discussed above. The results of the testing are shown in Table 8.

**Table 8**

| Durometer Hardness | | | | |
|---|---|---|---|---|
| Sample No. | Comparative Glove 3 | Comparative Glove 4 | Comparative Glove 5 | Inventive Glove 2 |
| | | | | |
| 1 | 54 | 62 | 57 | 54 |
| 2 | 55 | 60 | 57 | 52 |
| 3 | 54 | 61 | 56 | 54 |

(continued)

| Durometer Hardness | | | | |
|---|---|---|---|---|
| Sample No. | Comparative Glove 3 | Comparative Glove 4 | Comparative Glove 5 | Inventive Glove 2 |
| 4 | 54 | 62 | 57 | 53 |
| 5 | 54 | 63 | 56 | 52 |
| **Average** | 54 | 62 | 57 | 53 |

Inventive Glove 2 had a better average durometer hardness than Comparative Gloves 3-5. Compare durometer hardness of 53 of Inventive Glove 2 for the durometer hardnesses of 54, 57, and 62 for Comparative Gloves 3-5, respectively. It is also believed that durometer hardness is indicative of softness and that the lower the durometer hardness, the softer the glove. Thus, this is evidence that that Inventive Glove 2 was softer than Comparative Gloves 3-5.

Example 5

A test was conducted to compare the compressive strength readings of Comparative Gloves 3-5 and Inventive Glove 2. Inventive Glove 2 was the same as described in Example 4. Comparative Glove 3 was marketed by Ansell as its MICRO-TOUCH®ELITE® Product No. 3092. Comparative Glove 4 was marketed by Kimberly-Clark as its SAFESKIN®Synthetic Plus Product No. 50032. Comparative Glove 5 was marketed by Allegiance as its ESTEEM™ Product No. 8882. The compressive strength test was determined for Inventive Glove 2 and Comparative Gloves 3-5 in accordance with the procedure discussed above. The results of the testing are shown in Table 9.

**Table 9**

| Compressive Strength Test | | | | | | | | | | | | | |
|---|---|---|---|---|---|---|---|---|---|---|---|---|---|
| Product Type | Specimen | 1.25mm (0.050") Def. | | 1.905mm (0.075") Def. | | 2.54mm (0.100") Def. | | 3.175mm (0.125") Def. | | 3.81mm (0.150") Def. | | 4.445mm (0.175") Def. | |
| | No. | kg | (Lbs.) | kg | (Lbs.) | kg | (Lbs.) | kg | (Lbs.) | kg | (Lbs.) | kg | (Lbs.) |
| Comparative Glove 3 | 1 | 29,30 | 64,60 | 67,81 | 149,49 | 113,62 | 250,48 | 178,42 | 393,36 | 256,20 | 564,82 | 852,48 | 852,48 |
| | 2 | 26,86 | 59,21 | 65,13 | 143,59 | 109,11 | 240,55 | 174,01 | 383,62 | 266,43 | 587,39 | 815,48 | 815,48 |
| | 3 | 32,65 | 71,98 | 71,11 | 156,77 | 115,44 | 254,50 | 180,47 | 397,87 | 273,40 | 602,75 | 903,74 | 903,74 |
| | Average | 29,60 | 65,26 | 68,02 | 149,95 | 112,72 | 248,5 | 177,63 | 391,62 | 265,35 | 584,99 | 857,23 | 857,23 |
| Comparative Glove 4 | 1 | 33,52 | 73,69 | 72,78 | 160,46 | 114,35 | 252,11 | 170,69 | 376,32 | 256,60 | 565,71 | 871,76 | 871,76 |
| | 2 | 41,58 | 91,66 | 82,06 | 160,91 | 120,25 | 265,11 | 183,43 | 404,39 | 275,08 | 606,46 | 913,47 | 913,47 |
| | 3 | 40,46 | 89,20 | 82,67 | 182,26 | 119,08 | 262,52 | 185,36 | 408,66 | 289,10 | 637,36 | 954,83 | 954,83 |
| | Average | 38,51 | 84,91 | 79,17 | 174,54 | 117,89 | 259,91 | 179,83 | 396,46 | 273,60 | 603,18 | 913,35 | 913,35 |
| Comparative Glove 5 | 1 | 33,65 | 74,19 | 67,74 | 149,35 | 100,28 | 221,07 | 152,88 | 337,05 | 222,96 | 491,54 | 704,08 | 704,08 |
| | 2 | 32,42 | 71,47 | 66,49 | 146,58 | 97,38 | 214,68 | 154,84 | 341,36 | 228,78 | 504,37 | 816,91 | 816,91 |
| | 3o | 28,47 | 62,77 | 63,63 | 140,29 | 99,33 | 218,98 | 150,17 | 331,07 | 224,13 | 494,13 | 791,46 | 791,46 |
| | Average | 31,51 | 69,47 | 65,96 | 145,41 | 98,99 | 218,24 | 152,63 | 336,49 | 225,29 | 496,68 | 770,82 | 770,82 |
| Inventive Glove 2 | 1 | 30,75 | 67,79 | 57,33 | 126,39 | 92,08 | 203,00 | 134,16 | 295,78 | 207,30 | 457,02 | 711,46 | 711,46 |
| | 2 | 30,39 | 67,00 | 57,00 | 125,66 | 90,08 | 198,59 | 132,85 | 292,88 | 205,30 | 452,62 | 728,92 | 728,92 |
| | 3 | 29,21 | 64,39 | 55,13 | 121,54 | 88,84 | 195,85 | 129,84 | 286,25 | 200,87 | 442,84 | 680,39 | 680,39 |
| | Average | 30,12 | 66,40 | 56,49 | 124,53 | 90,33 | 199,15 | 132,28 | 291,64 | 204,49 | 450,83 | 706,92 | 706,92 |

Inventive Glove 2 had a better average compressive strength than Comparative Gloves 3-5. Compare, for example, average compressive strength at 0.100 inch of 199 for Inventive Glove 2 and average compressive strengths of 249,260 and 218 for Comparative Gloves3-5, respectively. It is also believed that compressive strength is indicative of softness and that the lower the compressive strength, the softer the glove. Thus, this is further evidence that Inventive Glove 2 was softer than Comparative Gloves 3-5.

Example 6

A test was conducted on a set of several commercial samples of Inventive Glove 2. Inventive Glove 2 was the same as described in Example 4. The tensile strength and the elongation of break were determined from 5 sample sets, in which 13 samples from each set were tested. The average tensile strength and elongation of break numbers for each set were recorded as "Set # 1-5"in Table 10. The average tensile strength and elongation of break numbers from Sets 1-5 were also included in, Table 10.

Table 10

| Set # | Tensile Strength ($M_{pa}$) of Inventive Glove 2 | Elongation of Break of Inventive Glove 2 |
| --- | --- | --- |
| 1 | 15.4 | 590 |
| 2 | 15.0 | 592 |
| 3 | 15.5 | 592 |
| 4 | 15.4 | 599 |
| 5 | 15.3 | 603 |
| Average | 15.3 | 595 |

As shown in Table 10, the tensile strength and elongation of break numbers of Inventive Glove 2 were desirable.

While particular embodiments and applications of the present invention have been illustrated and described, it is to be understood that the invention is not limited to the precise construction and compositions disclosed herein and that various modifications, changes, and variations may be apparent from the foregoing descriptions without departing from the scope of the invention as defined in the appended claims.

**Claims**

1. A disposable glove (10) **characterized in** comprising a first polyvinyl chloride resin having a degree of polymerization of from about 1,400 to about 1,700 as measured in accordance with JIS K 6721-77, a second polyvinyl chloride resin having a degree of polymerization of at least 1,750 as measured in accordance with JIS K 6721-77, a plasticizer and an elastomer.

2. The glove (10) of claim 1 further comprising a viscosity reducer.

3. The glove (10) of claim 2 further comprising a stabilizing agent.

4. The glove (10) of claim 1 wherein the glove comprises a ratio of the first polyvinyl chloride resin to the second polyvinyl chloride resin of from about 0.40 to about 0.75.

5. The glove (10) of claim 4 wherein the glove comprises a ratio of the first polyvinyl chloride resin to the second polyvinyl chloride resin of from about 0.55 to about 0.65.

6. The glove (10) of claim 1 wherein the plasticizer is diisononyl phthalate, dioctyl phthalate, diisodecyl phthalate, di (2-ethylhexyl) phthalate, diisodecyl phthalate, or combinations thereof.

7. The glove (10) of claim 1 wherein the plasticizer is diisononyl phthalate.

8. The glove (10) of claim 1 wherein the glove comprises a greater amount by weight of the plasticizer than the first polyvinyl chloride resin and wherein the glove comprises a greater amount by weight of the plasticizer than the second polyvinyl chloride resin.

9. The glove (10) of claim 8 wherein the glove comprises a greater total amount by weight of the first and second polyvinyl chloride resins than the amount by weight of the plasticizer.

10. The glove (10) of claim 1 wherein the second polyvinyl chloride resin has a degree of polymerization of from about 1,800 to about 2,200 as measured in accordance with JIS K 6721-77.

11. The glove (10) of claim 10 wherein the second polyvinyl chloride resin has a degree of polymerization of from about 1,900 to about 2,100 as measured in accordance with J IS K 6721-77.

12. A method of forming a disposable glove (10) **characterized in** comprising the acts of: providing a first polyvinyl chloride resin having a degree of polymerization of from about 1,400 to about 1,700 as measured in accordance with JIS K 6721-77, a second polyvinyl chloride resin having a degree of polymerization of at least 1,750 as measured in accordance with JIS K 6721-77, a plasticizer and an elastomer; mixing the first polyvinyl chloride, the second polyvinyl chloride, the plasticizer and the elastomer to form a mixture; dipping the mixture onto a glove-forming surface; and drying the mixture to form the glove.

13. The method of claim 12 further includes heating the mixture after the mixture is dipped.

14. The method of claim 12 further including providing a viscosity reducer and mixing the viscosity reducer with the first polyvinyl chloride, the second polyvinyl chloride, the plasticizer and the elastomer to form the mixture.

15. The method of claim 12 further including providing a stabilizing agent and mixing the stabilizing agent with the first polyvinyl chloride, the second polyvinyl chloride, the plasticizer and the elastomer to form the mixture.

16. The method of claim 12 wherein the glove (10) comprises a ratio of the first polyvinyl chloride resin to the second polyvinyl chloride resin of from about 0.40 to about 0.75.

17. The method of claim 16 wherein the glove (10) comprises a ratio of the first polyvinyl chloride resin to the second polyvinyl chloride resin of from about 0.55 to about 0.65.

18. The method of claim 12 wherein the plasticizer is diisononyl phthalate, dioctyl phthalate, diisodecyl phthalate, diisodecyl phthalate (DIDP) or combinations thereof.

19. The method of claim 12 wherein the plasticizer is diisononyl phthalate.

20. The method of claim 12 wherein the glove (10) comprises a greater amount by weight of the plasticizer than the first polyvinyl chloride resin and wherein the glove comprises a greater amount by weight of the plasticizer than the second polyvinyl chloride resin.

21. The method of claim 20 wherein the glove (10) comprises a greater total amount by weight of the first and second polyvinyl chloride resins than the amount by weight of the plasticizer.

**Patentansprüche**

1. Wegwerfhandschuh (10), **dadurch gekennzeichnet, dass** er ein erstes Polyvinylchloridharz mit einem Polymerisationsgrad von ungefähr 1400 bis ungefähr 1700, gemessen in Übereinstimmung mit JIS K 6721-77, ein zweites Polyvinylchloridharz mit einem Polymerisationsgrad von wenigstens 1750, gemessen in Übereinstimmung mit JIS K 6721-77, einen Weichmacher und ein Elastomer umfasst.

2. Handschuh (10) nach Anspruch 1, außerdem umfassend einen Viskositätserniedriger.

3. Handschuh (10) nach Anspruch 2, außerdem umfassend ein Stabilisierungsmittel.

4. Handschuh (10) nach Anspruch 1, wobei der Handschuh ein Verhältnis des ersten Polyvinylchloridharzes zu dem zweiten Polyvinylchloridharz von ungefähr 0,40 bis ungefähr 0,75 umfasst.

5. Handschuh (10) nach Anspruch 4, wobei der Handschuh ein Verhältnis des ersten Polyvinylchloridharzes zu dem zweiten Polyvinylchloridharz von ungefähr 0,55 bis ungefähr 0,65 umfasst.

6. Handschuh (10) nach Anspruch 1, wobei es sich bei dem Weichmacher um Diisononylphthalat, Dioctylphthalat, Diisodecylphthalat, Di(2-ethylhexyl)phthalat, Diisodecylphthalat oder Kombinationen davon handelt.

7. Handschuh (10) nach Anspruch 1, wobei es sich bei dem Weichmacher um Diisononylphthalat handelt.

8. Handschuh (10) nach Anspruch 1, wobei der Handschuh eine größere Menge, bezogen auf das Gewicht, von dem Weichmacher als von dem ersten Polyvinylchloridharz umfasst und wobei der Handschuh eine größere Menge, bezogen auf das Gewicht, von dem Weichmacher als von dem zweiten Polyvinylchloridharz umfasst.

9. Handschuh (10) nach Anspruch 8, wobei der Handschuh eine Gesamtmenge, bezogen auf das Gewicht, von dem ersten und zweiten Polyvinylchloridharz umfasst, die größer ist als die Menge, bezogen auf das Gewicht, von dem Weichmacher.

10. Handschuh (10) nach Anspruch 1, wobei das zweite Polyvinylchloridharz einen Polymerisationsgrad von ungefähr 1800 bis ungefähr 2200, gemessen in Übereinstimmung mit JIS K 6721-77, aufweist.

11. Handschuh (10) nach Anspruch 10, wobei das zweite Polyvinylchloridharz einen Polymerisationsgrad von ungefähr 1900 bis ungefähr 2100, gemessen in Übereinstimmung mit JIS K 6721-77, aufweist.

12. Verfahren zum Formen eines Wegwerfhandschuhs (10), **dadurch gekennzeichnet, dass** es die folgenden Vorgänge umfasst: Bereitstellen eines ersten Polyvinylchloridharzes mit einem Polymerisationsgrad von ungefähr 1400 bis ungefähr 1700, gemessen in Übereinstimmung mit JIS K 6721-77, eines zweiten Polyvinylchloridharzes mit einem Polymerisationsgrad von wenigstens 1750, gemessen in Übereinstimmung mit JIS K 6721-77, eines Weichmachers und eines Elastomers; das Vermischen des ersten Polyvinylchlorids, des zweiten Polyvinylchlorids, des Weichmachers und des Elastomers unter Bildung einer Mischung; das Aufbringen der Mischung mittels Eintauchen auf eine Handschuh-formende Oberfläche; und das Trocknen der Mischung, um den Handschuh zu bilden.

13. Verfahren nach Anspruch 12, außerdem umfassend das Erwärmen der Mischung, nachdem die Mischung mittels Eintauchen aufgebracht ist.

14. Verfahren nach Anspruch 12, außerdem umfassend das Bereitstellen eines Viskositätserniedrigers und das Vermischen des Viskositätserniedrigers mit dem ersten Polyvinylchlorid, dem zweiten Polyvinylchlorid, dem Weichmacher und dem Elastomer, um die Mischung zu bilden.

15. Verfahren nach Anspruch 12, außerdem umfassend das Bereitstellen eines Stabilisierungsmittels und das Vermischen des Stabilisierungsmittels mit dem ersten Polyvinylchlorid, dem zweiten Polyvinylchlorid, dem Weichmacher und dem Elastomer, um die Mischung zu bilden.

16. Verfahren nach Anspruch 12, wobei der Handschuh (10) ein Verhältnis des ersten Polyvinylchloridharzes zu dem zweiten Polyvinylchloridharz von ungefähr 0,40 bis ungefähr 0,75 umfasst.

17. Verfahren nach Anspruch 16, wobei der Handschuh (10) ein Verhältnis des ersten Polyvinylchloridharzes zu dem zweiten Polyvinylchloridharz von ungefähr 0,55 bis ungefähr 0,65 umfasst.

18. Verfahren nach Anspruch 12, wobei es sich bei dem Weichmacher um Diisononylphthalat, Dioctylphthalat, Düsodecylphthalat, Düsodecylphthalat (DIDP) oder Kombinationen davon handelt.

19. Verfahren nach Anspruch 12, wobei es sich bei dem Weichmacher um Diisononylphthalat handelt.

20. Verfahren nach Anspruch 12, wobei der Handschuh (10) eine größere Menge, bezogen auf das Gewicht, von dem Weichmacher als von dem ersten Polyvinylchloridharz umfasst und wobei der Handschuh eine größere Menge, bezogen auf das Gewicht, von dem Weichmacher als von dem zweiten Polyvinylchloridharz umfasst.

21. Verfahren nach Anspruch 20, wobei der Handschuh (10) eine Gesamtmenge, bezogen auf das Gewicht, von dem ersten und zweiten Polyvinylchloridharz umfasst, die größer ist als die Menge, bezogen auf das Gewicht, des Weichmachers.

**Revendications**

1. Gant jetable (10), **caractérisé en ce qu'**il comprend une première résine de poly(chlorure de vinyle) ayant un degré de polymérisation d'environ 1400 à environ 1700, de la manière mesurée suivant la norme JIS K 6721-77, une seconde résine de poly(chlorure de vinyle) ayant un degré de polymérisation d'au moins 1750, de la manière mesurée suivant la norme JIS K 6721-77, un plastifiant et un élastomère.

2. Gant (10) suivant la revendication 1, comprenant en outre un réducteur de viscosité.

3. Gant (10) suivant la revendication 2, comprenant en outre un agent stabilisant.

4. Gant (10) suivant la revendication 1, le gant comprenant un rapport de la première résine de poly(chlorure de vinyle) à la seconde résine de poly(chlorure de vinyle) d'environ 0,40 à environ 0,75.

5. Gant (10) suivant la revendication 4, le gant comprenant un rapport de la première résine de poly(chlorure de vinyle) à la seconde résine de poly(chlorure de vinyle) d'environ 0,55 à environ 0,65.

6. Gant (10) suivant la revendication 1, dans lequel le plastifiant est le phtalate de diisononyle, le phtalate de dioctyle, le phtalate de diisodécyle, le phtalate de di(2-éthylhexyle), le phtalate de diisodécyle ou leurs associations.

7. Gant (10) suivant la revendication 1, dans lequel le plastifiant est le phtalate de diisononyle.

8. Gant (10) suivant la revendication 1, le gant comprenant une quantité pondérale du plastifiant supérieure à celle de la première résine de poly(chlorure de vinyle) et le gant comprenant une quantité pondérale du plastifiant supérieure à celle de la seconde résine de poly(chlorure de vinyle).

9. Gant (10) suivant la revendication 8, le gant comprenant une quantité pondérale totale des première et seconde résines de poly(chlorure de vinyle) supérieure à la quantité pondérale du plastifiant.

10. Gant (10) suivant la revendication 1, dans lequel la seconde résine de poly(chlorure de vinyle) a un degré de polymérisation d'environ 1800 à environ 2200, de la manière mesurée suivant la norme JIS K 6721-77.

11. Gant (10) suivant la revendication 10, dans lequel la seconde résine de poly(chlorure de vinyle) a un degré de polymérisation d'environ 1900 à environ 2100, de la manière mesurée suivant la norme JIS K 6721-77.

12. Procédé pour former un gant jetable (10), **caractérisé en ce qu'**il comprend les opérations consistant à : fournir une première résine de poly(chlorure de vinyle) ayant un degré de polymérisation d'environ 1400 à environ 1700 de la manière mesurée suivant la norme JIS K 6721-77, une seconde résine de poly(chlorure de vinyle) ayant un degré de polymérisation d'au moins 1750 de la manière mesurée suivant la norme JIS K 6721-77, un plastifiant et un élastomère ; mélanger le premier poly(chlorure de vinyle), le second poly(chlorure de vinyle), le plastifiant et l'élastomère pour former un mélange ; appliquer le mélange sur une surface de formation de gant par immersion ; et sécher le mélange pour former le gant.

13. Procédé suivant la revendication 12, comprenant en outre le chauffage du mélange après avoir appliqué le mélange par immersion.

14. Procédé suivant la revendication 12, comprenant en outre l'étape consistant à fournir un réducteur de viscosité et à mélanger le réducteur de viscosité au premier poly(chlorure de vinyle), au second poly(chlorure de vinyle), au plastifiant et à l'élastomère pour former le mélange.

15. Procédé suivant la revendication 12, comprenant en outre l'étape consistant à fournir un agent stabilisant et à mélanger l'agent stabilisant au premier poly(chlorure de vinyle), au second poly(chlorure de vinyle), au plastifiant et à l'élastomère pour former le mélange.

**16.** Procédé suivant la revendication 12, dans lequel le gant (10) comprend un rapport de la première résine de poly (chlorure de vinyle) à la seconde résine de poly(chlorure de vinyle) d'environ 0,40 à environ 0,75.

**17.** Procédé suivant la revendication 16, dans lequel le gant (10) comprend un rapport de la première résine de poly (chlorure de vinyle) à la seconde résine de poly(chlorure de vinyle) d'environ 0,55 à environ 0,65.

**18.** Procédé suivant la revendication 12, dans lequel le plastifiant est le phtalate de diisononyle, le phtalate de dioctyle, le phtalate de diisodécyle, le phtalate de diisodécyle (DIDP) ou leurs associations.

**19.** Procédé suivant la revendication 12, dans lequel le plastifiant est le phtalate de diisononyle.

**20.** Procédé suivant la revendication 12, dans lequel le gant (10) comprend une quantité pondérale du plastifiant supérieure à celle de la première résine de poly(chlorure de vinyle) et dans lequel le gant comprend une quantité pondérale du plastifiant supérieure à celle de la seconde résine de poly(chlorure de vinyle).

**21.** Procédé suivant la revendication 20, dans lequel le gant (10) comprend une quantité pondérale totale des première et seconde résines de poly(chlorure de vinyle) supérieure à la quantité pondérale du plastifiant.

20

24

24

24

26

24

18

22

12

10

Fig.1

2

2

28

14

16

10

t

14

Fig.2

**EP 1 785 105 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4082862 A